# EUROPÄISCHE PATENTSCHRIFT

(11) **EP 2 701 841 B1**
(45) Veröffentlichungstag und Bekanntmachung des Hinweises auf die Patenterteilung: **17.06.2015**
(21) Anmeldenummer: 12716396.2
(22) Anmeldetag: 19.04.2012
(51) Int. Cl.: B01J 23/50, B01J 35/00, B01J 35/02, B01J 35/04, B01J 35/06, C07C 45/37, C07C 45/38, C07C 47/02, C07C 47/04

(54) **VERFAHREN ZUR OXIDATIVEN DEHYDRIERUNG VON METHANOL ZU FORMALDEHYD AN SILBERHALTIGEN GESTRICKEN**
PROCESS FOR OXIDATIVE DEHYDROGENATION OF METHANOL TO FORMALDEHYDE OVER SILVER-CONTAINING KNITS
PROCÉDÉ DE DÉSHYDROGÉNATION OXYDANTE DE MÉTHANOL EN FORMALDÉHYDE SUR DES TOILES CONTENANT DE L'ARGENT

(30) Priorität: 26.04.2011 EP 11163635
(43) Veröffentlichungstag der Anmeldung: 05.03.2014
(73) Patentinhaber: BASF SE, 67056 Ludwigshafen (DE)
(72) Erfinder: BALTES, Christian, 68623 Lampertheim (DE); KOLIOS, Grigorios, 67435 Neustadt (DE); RESCH, Peter, 67310 Hettenleidelheim (DE); WEGERLE, Ulrike, 67550 Worms (DE); MÄURER, Torsten, 67245 Lambsheim (DE)
(86) Internationale Anmeldenummer: PCT/EP2012/057190
(87) Internationale Veröffentlichungsnummer: WO 2012/146528

(56) Entgegenhaltungen:
- EP-A1- 0 597 454
- GB-A- 353 071
- US-A- 3 678 139

## Beschreibung

Die vorliegende Erfindung betrifft ein Verfahren zur Herstellung von Formaldehyd durch oxidative Dehydrierung von Methanol an einem Katalysatorformkörper welcher geformte Silber enthaltende Fasern und/oder Fäden enthält, dadurch gekennzeichnet, dass der mittlere Durchmesser bzw. die mittlere Diagonalenlänge eines im wesentlichen rechteckigen oder quadratischen Querschnitts dieser Silber enthaltenden Fasern und/oder Fäden im Bereich von 30 bis 70 µm und die Dichte des Katalysatorformkörpers im Bereich von 2 bis 4 g/m³ liegt.

Das Verfahren zur Herstellung von Formaldehyd durch Oxidation/Dehydrierung von Methanol an einem Silberkatalysator ist lange bekannt, siehe zum Beispiel Ullmann's Encyclopedia of Industrial Chemistry, 2005, Seiten.1 ff.

Der verwendete Silberkatalysator kann in diversen Formen eingesetzt werden. Zum Beispiel als körniges kristallines Silber, aber auch in Form von Silbernetzen oder Silbergaze (im Englischen auch als "silber gauze" bezeichnet).

US 4,076,754 (Du Pont) beschreibt ein zweistufiges Verfahren zur Herstellung von Formaldehyd aus Methanol, Luft und Wasser. Als Katalysator werden 40 Lagen (sheets) "20 mesh silver gauze" (Übereinandergelegte Silber-Netze mit einer Maschenweite von 1,25 mm) verwendet, welche aus einem Silberdraht hergestellt werden, der einen Durchmesser von 0,014 inch (entspricht 350 µm oder 0,35 mm) hat. Die Dichte oder der Hohlraumanteil der "silver gauze" wird in US 4,076,754 nicht offenbart.

DE 2829035 A1 (Heraeus) beschreibt einen Katalysator aus katalytisch aktiven Metallfasern, die aus Silber, Platin, Rhodium, Palladium oder einer Legierung auf Basis eines dieser Metalle bestehen, wobei die Metallfasern nach Art eines Nadelverbundwerkstoffs filzartig untereinander verbunden sind. Der Katalysator kann für die Ammoniakoxidation, die Blausäure- oder Formaldehydherstellung verwendet werden. Der Querschnitt einer bändchenförmigen Faser kann, rechteckig mit den Dimensionen 100 µm und 50 µm sein,die Länge kann zwischen 10 cm und 1 m liegen.
Die Dichte oder der Hohlraumanteil der verfilzten Metallfaserkörper wird nicht beschrieben.

DE 3047193 A1 (Johnson Matthey) beschreibt einen Katalysator aus Silber oder einer Silberlegierung. Der Katalysatorkörper wird im Schmelzspinnverfahren oder Schmelzextraktionsverfahren hergestellt. Es werden zum Beispiel aus einem Band von 1 bis 2 mm Breite und 50 bis 60 µm Dicke durch Krumpfen (Aufprägen eines Wellenprofils) und Schneiden etwa 1 cm lange, somit eher kurzfasrige, wellige Katalysatorkörper erhalten.

DE 3047193 A1 offenbart kein Geflecht, Gewirk, Filz oder ähnliches aus diesen Katalysatorkörpern.

US-A 3,678,139 beschreibt die oxidative Dehydrierung von Methanol zu Formaldehyd mit Luft. Das Verfahren arbeitet zweistufig katalytisch, wobei in der ersten Stufe 75 Lagen an übereinandergelegten Silberdrahtnetzen verwendet werden.

Obwohl die katalytische Oxidation/Dehydrierung von Alkoholen, zu Aldehyden, insbesondere Methanol zu Formaldehyd, schon lange bekannt ist, bleibt immer noch Raum für Verbesserungen, zum Beispiel die Erhöhung der Katalysatoraktivität, der Selektivität hinsichtlich Formaldehyd, vorteilhaft bei gleichbleibender Katalysatoraktivität, oder des Druckverlusts am Katalysator.

Eigene Untersuchungen zeigen, insbesondere bei der oxidativen Dehydrierung von Methanol zu Formaldehyd, dass faser- oder drahthaltige Strukturen nicht ohne weiteres als Katalysatoren eingesetzt werden können. Die Beschaffenheit der Katalysatorformkörper muss nämlich die Einstellung einer stabilen gezündeten Reaktionszone unter den Betriebsbedingungen des technischen Verfahrens beispielsweise der oxidativen Dehydrierung von Methanol zu Formaldehyd, ermöglichen. Jedoch offenbart der aktuelle Stand der Technik nicht die dafür erforderlichen Merkmale derartiger Katalysatorformkörper.

Aufgabe der vorliegenden Erfindung war es die Aldehydausbeute des Prozesses der oxidativen Dehydrierung von Methanol zu Formaldehyd durch den Einsatz eines Katalysatorformkörpers, der geformte Silber enthaltende Fasern und/oder Fäden enthält, zu verbessern.

Das erfindungsgemäße Verfahren wird für die Herstellung von Formaldehyd (Methanal) aus Methanol eingesetzt, wie im folgenden beschrieben.

Für das Methanol-Oxidationsverfahren geeignete Ausgangsstoffe sind reines Methanol, technisches Methanol, nach einem Hoch- oder Niederdruck-Verfahren hergestelltes Rohmethanol oder vorteilhaft deren Mischungen mit Wasser; die Methanolkonzentration der wässrigen Gemische in dem Ausgangsstoff beträgt zweckmäßigerweise 60 bis 95 Gew.-%, vorzugsweise 70 bis 90 Gew.-%. In einer vorteilhaften Ausführungsform wird Rohmethanol, das nach den in der DE-B-12 77 834, DE-C-12 35 881 und DE-C-11 36 318 beschriebenen Verfahren durch Abtrennung einer niedriger siedenden Fraktion bzw. durch Behandlung mit Oxidationsmitteln und/oder Alkalien gereinigt wurde, verwendet.

Das Methanol wird dem Reaktorraum in Dampfform zugeführt, vorteilhaft im Gemisch mit Wasserdampf und gegebenenfalls mit einem Inertgas. Als Inertgas kommt für das Verfahren beispielsweise Stickstoff in Betracht.

Als oxidierendes Agens lassen sich sowohl reiner Sauerstoff als auch vorzugsweise Sauerstoffenthaltende Gase, insbesondere Luft, verwenden. Sauerstoff und Methanol werden zweckmäßigerweise im Molverhältnis von 0,25 bis 0,6, insbesondere von 0,35 bis 0,5 Mol Sauerstoff pro Mol Methanol verwendet. Vorzugsweise beträgt die Gesamtmenge an Wasserdampf nicht mehr als 3,0, vorteilhaft 0,67 bis 1,75 mol pro Mol Methanol.

Im technischen Prozess der Formaldehydsynthese wird die oben beschriebene Reaktionsmischung im allgemeinen mit einer Temperatur zwischen 50 und 200°C sowie üblicherweise bei einem Absolutdruck zwischen 1 und 2 bar in den Reaktor eingeleitet.

Die genannten Ausgangsstoffe werden dann üblicherweise in eine Zone oder mehrere Zonen geleitet, in welcher oder welchen sich der erfindungsgemäße Katalysatorformkörper befindet.

Der erfindungsgemäße Katalysatorformkörper ist ein dreidimensionales Gebilde, welches erhältlich ist durch dreidimensionales Verformen und/oder Anordnen im Raum von silberhaltigen Fasern oder silberhaltigen Fäden.

Die silberhaltigen Fasern oder Fäden enthalten Silber in einer Menge im Bereich von 50 bis 100 Gew.-%, vorzugsweise bis 100 Gew.-%, besonders bevorzugt 98 bis 100 Gew.-%, und weitere Metalle der 10ten oder 11ten Gruppe des Periodensystems der Elemente, vorzugsweise Metalle ausgewählt aus der Gruppe bestehend aus Kupfer, Palladium, Titan im Bereich von 0 bis 50 Gew.-%, vorzugsweise 0 bis 10 Gew-%, besonders bevorzugt 0 bis 2 Gew.-%.

Gut geeignete silberhaltige Fasern oder Fäden enthalten zu praktisch 100 Gew.-% Silber.

Silberhaltige, erfindungsgemäße Fasern haben in der Regel eine Länge im Bereich von ca. 1 mm bis 100 mm, silberhaltige erfindungsgemäße Fäden können theoretisch endlos sein, praktisch haben sie in der Regel eine Länge von wenigen Zentimetern bis mehrere Kilometer.

Der mittlere Durchmesser (für im Wesentlichen runden Querschnitt) bzw. die mittlere Diagonalenlänge (für im Wesentlichen rechteckigen bzw. quadratischen Querschnitt) der silberhaltigen Fasern oder Fäden liegt im Bereich von 30 bis 70 µm.

Der mittlere Durchmesser bzw. mittlere Diagonallänge wird mit der Methode nach DIN ISO 4782 "Nenndrahtdurchmesser für Siebgewebe" bestimmt.

Silberhaltige Fasern oder Fäden sind dem Fachmann bekannt, kommerziell erhältlich und werden beispielsweise als elektrisches Leitermaterial, in hochwertigen Textilien oder in korrosionsbeständigen, sensorischen Anwendungen (z.B. pH-Wert-Bestimmung) eingesetzt.

Das dreidimensionale Verformen und/oder Anordnen der silberhaltigen Fasern oder Fäden im Raum kann ungeordnet oder geordnet erfolgen.

Das ungeordnete Verformen und/oder Anordnen der erfindungsgemäßen silberhaltigen Fasern oder vorzugsweise erfindunggemäßen silberhaltigen Fäden führt üblicherweise zu sogenannten Knäueln. Sie können beispielsweise dadurch erzeugt werden, dass Fasern oder Drähte zu einem statistisch ungleichmäßig angeordneten Knäul gepackt und anschließend mit verschiedenen Drücken auf die gewünschte Knäuel-Dichte bzw. den gewünschten Holraumamteil im Knäuel weiter komprimiert werden.
In derartigen Knäueln sind die erfindungsgemäßen silberhaltigen Fasern oder Fäden unregelmäßig im Raum angeordnet und können auch filzartig miteinander verhakt sein und dadurch beispielsweise ihre besondere mechanische Stabilität beziehen. Derartige Knäuel werden im Folgenden auch "erfindungsgemäße silberhaltige Knäuel" genannt.

Das geordnete Verformen und/oder Anordnen der silberhaltigen Fasern oder Fäden führt zu im Wesentlichen regelmäßigen und geordneten Strukturen mit sich periodisch wiederholenden Elementarzellen, zum Beispiel Maschen oder Löchern. Gut geeignete Verfahren zum geordneten Verformen und/oder Anordnen der silberhaltigen Fasern oder vorzugsweise Fäden im Raum ist das Verstricken oder Weben oder dergleichen und anschließende Verdichten.

Gut geeignete geordneten Strukturen aus silberhaltigen Fasern oder vorzugsweise silberhaltigen Fäden sind sogenannte Gestricke oder Netze, beispielsweise mit einer Maschenweite im Bereich von 300 bis 50 mesh (80 µm bis 500 µm], vorzugsweise im Bereich von 300 bis 100 mesh (80 µm bis 250 µm). Diese Gestricke oder Netze werden im Folgenden auch "erfindungsgemäße silberhaltige Gestricke" genannt.

Die erfindungsgemäßen silberhaltigen Gestricke oder erfindungsgemäßen silberhaltigen Knäuel haben in der Regel eine Dichte im Bereich von 2 bis 4 g/cm g/cm³, vorzugsweise im Bereich von 3 bis 4 g/cm³.

Die Dichte korrespondiert in der Regel mit einem Hohlraumanteil der erfindungsgemäßen silberhaltigen Gestricke oder erfindungsgemäßen silberhaltigen Knäuel im Bereich von 60 bis 80 %, vorzugsweise im Bereich von 60 bis 75 %.

Ein Hohlraumanteil von mehr als 80 % ist nachteilig. Der erfindungsgemäße Hohlraumanteil der erfindungsgemäßen silberhaltigen Gestricke oder erfindungsgemäßen silberhaltigen Knäuel ist auch deswegen vorteilhaft um ein "Zünden" der katalytischen Oxidation/Dehydrierung des Methanols bei möglichst niedrigen Temperaturen, beispielsweise 350 °C und weniger, vorteilhaft im Bereich von 200 bis 350 °C, zu gewährleisten. Üblicherweise wird das erfindungsgemäße silberhaltige Gestrick oder das erfindungsgemäße silberhaltige Knäuel vorgeheizt bis die Reaktion (oxidative Dehydrierung des Methanols zu Formaldehyd) anspringt. Danach unterhält sich die genannte Reaktion unter adiabatischen Bedingungen in der Regel selbst.

Die oben genannte Dichte und der Hohlraumanteil der erfindungsgemäßen silberhaltigen Gestricke oder erfindungsgemäße silberhaltigen Knäuel wird mit der Methode wie folgt bestimmt:

Ein Körper bekannter Geometrie wird gewogen. Das Verhältnis seines Gewichtes zum von ihm eingenommenen Volumen bestimmt die Dichte. Das Verhältnis zum Gewicht eines geometrisch gleichen massiven Körpers aus demselben Material definiert den Hohlraumanteil. Der erfindungsgemäße Katalysatorformkörper kann in mannigfaltiger Raumform vorliegen.

Beispielsweise können die, den erfindungsgemäße Katalysatorformkörper bildenden erfindungsgemäßen silberhaltigen Knäuel oder vorzugsweise die erfindungsgemäßen silberhaltigen Gestricke als Matten oder Scheiben, also als flächige Gebilde, deren Längen und Breiten um ein vielfaches größer sind als ihre Höhen vorliegen. Gegebenenfalls können mehrere Formkörper übereinander gestapelt oder segmentartig zusammengesteckt vorliegen.

Beispielsweise können die, den erfindungsgemäße Katalysatorformkörper bildenden erfindungsgemäßen silberhaltigen Knäuel oder vorzugsweise die erfindungsgemäßen silberhaltigen Gestricke aber auch als Raschig-Ringe und/oder als Wendeln vorliegen.

Die absoluten Abmessungen der erfindungsgemäßen Katalysatorformkörper richten sich in der Regel nach den Dimensionen des Reaktors in welchen der Katalysatorformkörper angewendet wird.

Beispielhafte Dimensionen der erfindungsgemäßen Katalysatorformkörper sind im Bereich von 120 bis 30 cm Länge, im Bereich von 50 bis 10 cm Breite und im Bereich von 1 bis 10 cm, vorzugsweise im Bereich von 2 bis 4 cm Höhe.

Die geometrische Gestalt des erfindungsgemäßen Katalysatorformkörpers ist in der Regel variabel.

Bevorzugt sind rechteckige, bzw. quaderförmige oder runde bzw. rundscheibenförmige oder zylindrische Katalysatorformkörper oben genannter Dimensionen, wobei der Durchmesser der runden Katalysatorformkörper beispielsweise im Bereich von 2 cm bis 300 cm, vorzugsweise im Bereich von 25 cm bis 300 cm und besonders bevorzugt im Bereich von 50 cm bis 300 cm liegt.

Üblicherweise wird der Katalysator-Formkörper im Reaktionsraum, in welchem die oben genannten Ausgangsstoffe, beispielsweise Alkohol, wie Methanol, sauerstoffhaltiges Gas, umgesetzt werden, auf einer Trägervorrichtung ruhend eingesetzt.

Derartige Trägervorrichtungen sind bekannt, es sind beispielsweise Roste, Körbe oder Lochplatten oder stabile Netze aus diversen Materialien, vorzugsweise aus Metallen, beispielsweise Edelstahl oder Silber.

Der erfindungsgemäße Katalysatorformkörper kann als einziger katalytisch aktiver Bestandteil in der Reaktionszone in welcher die oben genannten Ausgangsstoffe bzw. Stoffströme, die Methanol, Sauerstoff und Wasser enthalten, eingesetzt werden, vorliegen. Er kann aber auch im Beisein von granulären Silberkatalysatoren und/oder anderen Katalysatoren für die oxidative Dehydrierung von Alkoholen zu Aldehyden vorliegen.

Beispielsweise kann eine Schichtstruktur erfindungsgemäßer Katalysatorformkörper // granulärer Silberkatalysatoren vorliegen.

Es können auch mehrere Reaktionszonen, in welchen die oben genannten Ausgangsstoffe bzw. Stoffströme, beispielsweise Alkohol, wie Methanol, sauerstoffhaltiges Gas, eingesetzt werden und die den erfindungsgemäßen Katalysator enthalten, in "Reihe geschaltet" vorliegen. Diese Reihenschaltung kann in einem Reaktor oder in einer Reaktorkaskade verwirklicht werden.

Das Verfahren wird im Übrigen in an sich bekannter Weise durchgeführt, indem man z. B. ein Gasgemisch aus Methanoldampf, Luft, gegebenenfalls Inertgas und zweckmäßig Wasserdampf in vorgenannten Mengen bei Temperaturen von etwas 550 bis 750 °C, insbesondere 595 bis 710 °C, durch die Reaktionszone oder Reaktionszonen die den erfindungsgemäßen Katalysator enthalten leitet. Das Verfahren wird im Allgemeinen bei einem Absolutdruck zwischen 0,5 und 2 bar, vorzugsweise zwischen 1,2 und 1,8 bar, kontinuierlich durchgeführt. Dabei ist es vorteilhaft, die die Katalysatorzone verlassenden Reaktionsgase innerhalb kurzer Zeit abzukühlen, z. B. auf Temperaturen von 50 bis 350 °C. Das abgekühlte Gasgemisch wird dann zweckmäßigerweise einem Absorptionssturm zugeführt, in welchem der Formaldehyd mit Wasser, vorteilhaft im Gegenstrom, aus dem Gasgemisch gewaschen wird.

Das Verfahren ist darüber hinaus in Ullmann's Encyclopedia of Industrial Chemistry, 2005, Seiten1 ff. näher beschrieben.

Die Vorteile des erfindungsgemäßen Verfahrens sind insbesondere:
- Eine verbesserte Ausbeute an Formaldehyd, im Vergleich zu den herkömmlichen Katalysatoren (höhere Selektivitäten zu beispielsweise Formaldehyd und beispielsweise vergleichbare Methanolumsätze bei geringerer Katalysatormasse).
- Eine verbesserte Gleichmäßigkeit der Katalysatorpackung hinsichtlich Schichtdicke und Materialdichte.
- Die Möglichkeit der Einflussnahme auf die katalytischen Eigenschaften des erfindungsgemäße Katalysatorformkörper durch die gezielte Einstellung der Geometrie des Formkörpers, insbesondere der Draht-/FaserStruktur, den Durchmesser betreffend, und die Dichte des Formkörpers.

Überraschenderweise ergaben sich Zusammenhänge zwischen den geometrischen und strukturellen Parametern der Katalysatorformkörper und der gesteigerten chemischen Produktivität. Hierbei wurde beobachtet, dass zum Beispiel bei der oxidativen Dehydrierung von Methanol mit abnehmendem Drahtdurchmesser die Formaldehydausbeute im einfachen Durchgang durch das Katalysatorbett ansteigt. Weiterhin korreliert ein vorteilhaftes Zündverhalten des Katalysatorformkörpers mit dessen Packungsdichte.

### Beispiele

### Beispiel 1

Ein gasförmiges Wasser-/Methanolgemisch (molares Verhältnis von Wasser/Methanol: 1,0) wurde mit Luft (140 Nl/h)und Stickstoff (50 Nl/h) in dem Maße vermischt, dass das molare Verhältnis von Methanol zu Sauerstoff 2,5 betrug. Dieses Gemisch wurde in einem dem Reaktor vorgelagerten Vorheizer auf 140 °C erwärmt und anschließend über einen Silbergestrick-Katalysator geleitet. Dieser Katalysator bestand aus einem zylindrischen Formkörper mit einer Höhe von 10 mm und einem Durchmesser von 20 mm. Der Formkörper bestand aus verpresster Silberwolle mit einem Faserdurchmesser von 0,05 mm (Dichte des Formkörpers: 3 g/cm³; Hohlraumanteil: 75 %). Die Versuche wurden in adiabater Fahrweise in einem Quarzglasreaktor mit einem Innendurchmesser von 20 mm durchgeführt. Die Adiabasie des Reaktors wurde durch passive Isolation erreicht, und verzichtet komplett auf eine Kompensationssheizung. Um eine Zündung der adiabat ausgeführten Reaktion am Silberkatalysator zu gewährleisten, wurde das Methanol/Wasser/Luft/Stickstoff-Gemisch auf 300 °C erwärmt, wobei bei diesen Temperaturen das molare Verhältnis Methanol/Sauerstoff 7:1 betrug und eine Stickstoffdosierung von 300 Nl/h erfolgt. Die adiabate Zündung erfolgte bei 300 °C. Anschließend wurde schrittweise die oben genannte Zusammensetzung von Wasser/Methanol/Luft/Stickstoff dosiert. Wenn Dosierung und Vorheizertemperatur eingestellt waren wie oben beschrieben, erreichte das Katalysatorbett bei gezündeter adiabater Reaktion Temperaturen von 595 °C. Es wurde eine Gasbelastung des Katalysators von 95.000 h⁻¹ erreicht. Das aus dem Katalysatorbett austretende Produktgemisch wurde an einem Wärmetauscher auf 120 °C gekühlt. Die Zusammensetzung des Produktgemischs wurde durch einen Gaschromatographen analysiert. Unter den genannten Bedingungen wurden ein Methanolumsatz von 99 % und eine Formaldehydselektivität von 90 % erreicht. Ein herkömmlich genutzter elektrolytisch hergestellter Silbergranulatkatalysator (Fraktionsgröße 0,5 bis 2 mm) erreichte bei einem Methanolumsatz von 99 % eine Formaldehydselektivität von 87 %.

### Beispiel 2 (Vergleichsbeispiel)

Die Versuchsführung hinsichtlich der Edukt-Dosierung und Katalysator-Zündung entsprach Beispiel 1, soweit nicht anders beschrieben. Als Katalysator wurde ein dreidimensionaler zylindrischer Formkörper aus verpressten Silbernetzen verwendet. Der Durchmesser des Silberdrahtes betrug 0,076 mm. Die Höhe des Katalysatorformkörpers betrug 20 mm, der Durchmesser lag bei 20 mm. Unter den genannten Bedingungen wurden ein Methanolumsatz von 98% und eine Formaldehydselektivität von 90 % erreicht. Ein herkömmlich genutzter elektrolytisch hergestellter Silbergranulatkatalysator (Fraktionsgröße 0,5 bis 2 mm) erreichte bei einem Methanolumsatz von 98 % eine Formaldehydselektivität von 87 %.

### Beispiel 3 (Vergleichsbeispiel)

Die Versuchsführung hinsichtlich der Edukt-Dosierung und Katalysator-Zündung entsprach Beispiel 1, soweit nicht anders beschrieben. Als Katalysator wurde ein dreidimensionaler zylindrischer Formkörper aus einem verstrickten und anschließend gepressten Silberdraht verwendet. Der Durchmesser des Silberdrahtes betrug 0,1 mm. Die Dichte des gepressten Gestricks betrug 3 g/cm³ Die Höhe des Katalysatorformkörpers betrug 10 mm, der Durchmesser lag bei 20 mm. Unter den genannten Bedingungen wurden ein Methanolumsatz von 96% und eine Formaldehydselektivität von 91 % erreicht. Ein herkömmlich genutzter elektrolytisch hergestellter Silbergranulatkatalysator (Fraktionsgröße 0,5 bis 2 mm) erreichte bei einem Methanolumsatz von 96 % eine Formaldehydselektivität von 90 %.

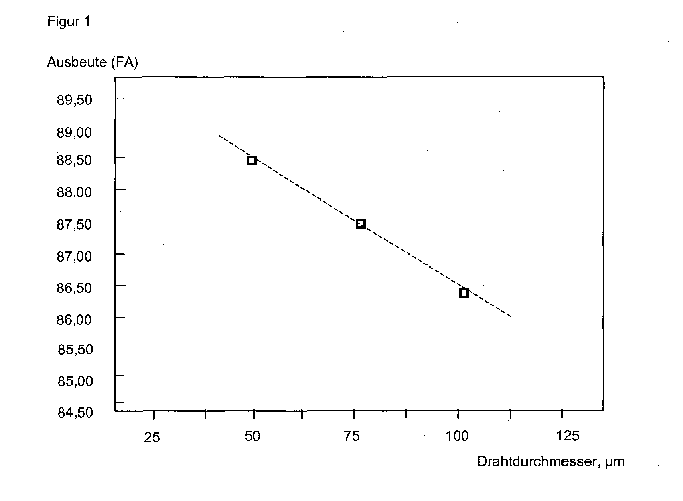

Parameter aus den Beispielen 1 bis 3 sind in folgender Figur 1 dargestellt.

Figur 1: Abhängigkeit der Leistung des Katalysators (Ausbeute an Formaldehyd, bezogen auf Methanol) vom Drahtdurchmesser des Drahtes, aus dem der katalytische Formkörper hergestellt wurde. Alle Formkörper weisen das gleiche Volumen und die gleiche Dichte auf. Die Reaktionsbedingungen sind identisch.

## Patentansprüche

1. Verfahren zur Herstellung von Formaldehyd durch oxidative Dehydrierung von Methanol an einem Katalysatorformkörper welcher durch dreidimensionales Verformen und/oder Anordnen im Raum von Silber-enthaltenden Fasern und/oder Fäden erhältlich ist, **dadurch gekennzeichnet, dass** der mittlere Durchmesser bzw. die mittlere Diagonalenlänge eines im wesentlichen rechteckigen oder quadratischen Querschnitts dieser Silber enthaltenden Fasern und/oder Fäden im Bereich von 30 bis 70 µm, die Dichte des Katalysatorformkörpers im Bereich von 2 bis 4 g/cm³ liegt.

2. Verfahren gemäß Anspruch 1, **dadurch gekennzeichnet, dass** das dreidimensionale Verformen und/oder Anordnen im Raum ungeordnet oder geordnet erfolgt.

3. Verfahren gemäß Anspruch 1 bis 2, **dadurch gekennzeichnet, dass** die geformten Silberenthaltenden Fasern und/oder Silber-enthaltende Fäden ungeordnet und in Form von Knäueln vorliegen.

4. Verfahren gemäß Anspruch 1 bis 2, **dadurch gekennzeichnet, dass** die geformten Silberenthaltenden Fasern und/oder Silber-enthaltende Fäden geordnet und in Form von Gestricken oder Netzen vorliegen.

## Claims

1. A process for producing formaldehyde by oxidative dehydrogenation of methanol over a shaped catalyst body obtainable by three-dimensional shaping and/or arranging in space of silver-containing fibers and/or threads, wherein the average diameter or the average diagonal length of an essentially rectangular or square cross section of these silver-containing fibers and/or threads is in the range from 30 µm to 70 µm and the density of the shaped catalyst body is in the range from 2 to 4 g/cm³.

2. The process according to claim 1 wherein the three-dimensional shaping and/or arranging in space is effected with or without order.

3. The process according to claim 1 or 2 wherein the shaped silver-containing fibers and/or silver-containing threads are present without order and in the form of clews.

4. The process according to claim 1 or 2 wherein the shaped silver-containing fibers and/or silver-containing threads are present with order and in the form of knits or nets.

## Revendications

1. Procédé de fabrication de formaldéhyde par déshydrogénation oxydante de méthanol sur un corps moulé catalytique qui peut être obtenu par façonnage tridimensionnel et/ou agencement dans l'espace de fibres et/ou de fils contenant de l'argent, **caractérisé en ce que** le diamètre moyen ou la longueur diagonale moyenne d'une section essentiellement rectangulaire ou carrée de ces fibres et/ou fils contenant de l'argent se situe dans la plage allant de 30 à 70 µm, la densité du corps moulé catalytique se situe dans la plage allant de 2 à 4 g/cm³.

2. Procédé selon la revendication 1, **caractérisé en ce que** le façonnage tridimensionnel et/ou l'agencement dans l'espace a lieu de manière désordonnée ou ordonnée.

3. Procédé selon les revendications 1 à 2, **caractérisé en ce que** les fibres contenant de l'argent et/ou les fils contenant de l'argent façonnés se présentent sous forme désordonnée et sous la forme de pelotes.

4. Procédé selon les revendications 1 à 2, **caractérisé en ce que** les fibres contenant de l'argent et/ou les fils contenant de l'argent façonnés se présentent sous forme ordonnée et sous la forme de tricots ou de filets.
